# EUROPEAN PATENT APPLICATION

(11) **EP 4 671 755 A1**
(43) Date of publication of application: **31.12.2025**
(21) Application number: 24184888.6
(22) Date of filing: 27.06.2024
(51) Int. Cl.: G01N 33/44, G01M 5/00

(54) **METHOD, SYSTEM AND ARRANGEMENT FOR MONITORING A MECHANICAL PROPERTY OF AN ELASTOMERIC COMPONENT**

(71) Applicant: Dätwyler Schweiz AG, 6467 Schattdorf (CH)
(72) Inventor: Ahaggach, Yassine, 8047 Zurich (CH); Soddemann, Matthias, 6467 Schattdorf (CH); Lamkharbech, Yassine, 6460 Altdorf (CH); Vrijens, Ronny Prosper Elisabeth, 3721 Vliermaalroot (BE)
(74) Representative: Prins Intellectual Property AG

(57) **Abstract**

A method for monitoring the state of health and/or correct functioning of an elastomeric component, the method comprising the steps of: (a.) providing the elastomeric component, the elastomeric component comprising a matrix of elastomer material and homogenously dispersed magnetic particles; (b.) providing a magnetic sensor element for measuring magnetic properties of the elastomeric component with the dispersed magnetic particles, wherein the sensor is positioned in or near the elastomeric component; (c.) measuring magnetic properties of the elastomeric component with the dispersed magnetic particles; (d.) based on the measurements of the magnetic properties of step c.: (i.) determining mechanical properties of the elastomeric component related to strain of the elastomer matrix, a temperature effect on magnetism influenced by the magnetic particles or structural changes of the magnetic particles within the elastomer matrix due to humidity; (ii.) correlating the mechanical properties with the state of health and/or the proper functioning of the elastomeric component.

## Description

### Technical Field

The invention relates to a method for monitoring the state of health (SoH) and/or correct functioning of an elastomeric component in-situ.

### Technical Background

Elastomeric components are used in a wide variety of applications across multiple industries due to their unique properties such as flexibility, resilience, and durability. Applications where elastomeric components are utilized are seals, gaskets or membranes in sealing systems such as pumps, syringes, valves; hoses and tubing; suspension components (bushings, mounts to absorb vibrations and shocks), insulation components (thermal insulation), any many more.

In most applications it is of utmost importance that the actual state of health of an elastomeric component can be determined in order to replace the component before its failure. Nowadays, this is mainly done by periodic inspections and/or scheduled replacement of the components often well below its possible service life to avoid the risk of unexpected failure of the component.

Elastomeric components comprising embedded sensors such as strain gauges or pressure sensors are known to measure forces applied to the elastomeric components in real-time. However, the disadvantages are a limited coverage, high complexity in embedding the sensors, potential fragility of the sensor, and local impact on the properties of the elastomeric component where the sensor is embedded. Furthermore, it is not possible to determine mechanical properties of the elastomeric component, in particular of the matrix of elastomer material.

Radio-frequency techniques, ultrasound-based methods (such as pulse-echo or through-transmission) and light-based methods (such as infrared thermography or laser vibrometry) are used to monitor elastomeric components, to detect internal defects and provide information on mechanical properties. However, disadvantages of these methods are a limited penetration depth, in some cases only indirect measurement of the elastomeric properties, a requirement of access to the component surface, a complex interpretation of the signals, and they can often only be used for intermittent monitoring.

There is a need for a non-invasive method to monitor the performance and the state of health (also non-abrasive), and to predict the remaining useful life of elastomeric components.

### Summary of the Invention

It is an objective of the invention to provide a method and a system capable of non-invasive, in-situ monitoring of state of health, wear, and performance of an elastomeric component over time with minimal impact on the elastomeric component.

At least one of the objectives of the present invention is achieved by a method for monitoring the state of health and/or correct functioning of an elastomeric component in-situ according to claim 1, a system according to claim 7 and an arrangement according to claim 9.

The method for monitoring the state of health and/or correct functioning of an elastomeric component comprises the steps of: (a.) providing the elastomeric component, the elastomeric component comprising a matrix of elastomer material and homogenously dispersed magnetic particles; (b.) providing a magnetic sensor element for measuring magnetic properties of the elastomeric component with the dispersed magnetic particles, wherein the sensor is positioned in or near the elastomeric component; (c.) measuring magnetic properties of the elastomeric component with the dispersed magnetic particles; (d.) based on the measurements of the magnetic properties of step c: (i.) determining mechanical properties of the elastomeric component related to strain of the elastomer matrix, a temperature effect on magnetism influenced by the magnetic particles or structural changes of the magnetic particles within the elastomer matrix due to humidity; (ii.) correlating the mechanical properties with the state of health and/or the proper functioning of the elastomeric component.

A measurement of the magnetic properties of the elastomeric component can be achieved in two ways.

Firstly, the dispersed magnetic particles may be permanently magnetized to create a remanent magnetic field in the elastomeric component and the sensor element may measure the remanent magnetic field of the elastomeric component.

Secondly, the magnetic sensor element (i.e. a coil) may create a magnetic field and measure the interactions of the magnetic particles with the generated magnetic field (e.g. a change in magnetic flux or inductance). The magnetic field may be pulsed for continuous measurements.

A measurement may be compared to reference magnetic properties to determine a state of health. A measurement can also refer to changes in magnetic properties, which relate to changes in mechanical properties. The measurements may take place over a predetermined period of time to monitor changes in the magnetic properties. The measurements may typically occur at defined time intervals. The resolution of the measurement depends on the length of the time intervals. The measurements may record short-time changes in mechanical properties (e.g. in dynamic systems such as a pump) or long-time changes in mechanical properties (e.g. in static systems such as a bearing).

The inventors observed that the remanent magnetic field changes proportionally not only to the state of deformation (e.g. increase of remanence with compression) but also temperature (decrease of remanence from increase of temperature) or when the structure of the magnetic particles within the elastomer matrix changes due to humidity (e.g. corrosion of the magnetic particles). They realized that the distribution and motion of magnetic particles inside a defined elastomer volume will follow changes in the elastomer matrix. This effect of coupled magneto-mechanical behaviour can be used to determine relative changes in mechanical properties of the elastomeric component in-situ, i.e. in a device for the intended and functional arrangement of the elastomeric component. Thus, the method can measure mechanical properties and stress-strain behaviour of the elastomeric component resulting from the elastomer matrix to which the magnetic particles are bonded. Therefore, these behaviours backpropagate to the motion and position of the magnetic particles, which in turn influence the observable magnetic field.

Mechanical properties that can be correlated to changes in the magnetic properties of the elastomeric component are:
1. Elasticity: The ability to undergo significant deformation under stress and return to their original shape upon removal of the stress. This is characterized by high strain at low stress levels and its return to the original state after stress is released. A change in the remanent magnetic field under stress and a return to the original state upon removal of the stress is coupled to the deformation of the elastomeric component and relates to its elasticity in-situ. Changes in elasticity (sudden or over a long time) can be observed and correlated to the state of health and/or correct functioning of the elastomeric component in-situ.
2. Compression Set: The degree to which an elastomer does not return to its original shape after prolonged compressive stresses. It is a measure of the permanent deformation remaining after the material has been compressed and the stress removed. The remanent magnetic field of the elastomeric component directly relates to the deformation of the elastomeric component. Therefore, permanent deformation may be observed comparing the remanent magnetic field before and after prolonged compressive stresses.
3. Resilience (Rebound): The ability of an elastomer to absorb energy when deformed and release it upon unloading. High resilience indicates efficient energy return and less hysteresis loss. By comparing the remanent magnetic field during loading and unloading cycles of the elastomeric component, the resilience of the elastomeric component can be observed. A loss or sharp decrease in resilience indicates a bad state of health of the elastomeric component.
4. Fatigue / fatigue resistance: The ability of an elastomer to withstand repeated cycles of deformation without cracking or losing its mechanical properties. Fatigue results from a change in the matrix of the elastomeric component which results in changes of other mechanical properties. These changes can be observed by monitoring the remanent magnetic field of the elastomeric component created by the magnetic particles, because the particles are bound to the matrix. A high degree in fatigue indicates a bad state of health of the elastomeric component.
5. Creep / creep resistance: The resistance to gradual deformation under a constant stress over a long period. Elastomers with low creep resistance will deform significantly over time under a constant load. The deformation of the elastomeric component over time under a constant load can be measured by the remanent magnetic field of the elastomeric component. Reaching a predetermined threshold indicates a bad state of health of the elastomeric component.
6. Temperature Stability: The ability of an elastomer to retain its mechanical properties over a range of temperatures. Some elastomers are designed to perform well at high temperatures, while others are tailored for low-temperature applications. When correlating the measured remanent magnetic field with temperature measurements, the temperature stability of the elastomeric component may be monitored.
7. Chemical Resistance: The resistance of an elastomer to degradation by chemicals such as oils, solvents, acids, and bases. This property is crucial for elastomers used in harsh chemical environments. Even chemical resistance may be observed as degradation of the elastomeric component leading to a change of the remanent magnetic field.
8. Mullins Effect: The Mullins Effect is a stress-softening phenomenon observed in filled rubbers where the stress-strain curve on the first loading cycle is higher than on the subsequent cycles, even if the strain does not exceed the maximum of the first cycle. It is related to the breakage of weak bonds or the rearrangement of the filler network within the material. A relative shift of measured stress-strain curves is observable via the measurement of the remanent magnetic field and can be correlated with the Mullins Effect.
9. Hysteresis: Elastomers loading and unloading curves differ. The area between these curves represents energy lost as heat due to internal friction out of the materials viscoelasticity. Hysteresis is observable by measuring the remanent magnetic field of the elastomeric component during loading and unloading cycles. A significant change in hysteresis indicates a change in the structure of the elastomeric matrix. Reaching a predetermined threshold indicates a bad state of health of the elastomeric component.
10. Stress Relaxation: If an elastomer is deformed and then held at a constant strain, the stress required to maintain that strain decreases over time due to the internal structures of the material rearranging overtime to reduce the stress. The effect relates to the compression set and the resilience of the elastomeric component and can be observed by monitoring the remanent magnetic field of the elastomeric component.
11. Frequency Dependence: Elastomers show a dependence on the frequency of applied loads. Their stiffness and damping characteristics can change with the rate at which they are deformed, which is related to the time-dependent nature of viscoelastic materials. Stiffness and damping characteristic relate to the elasticity of the elastomeric component, which can be measured by monitoring the remanent magnetic field of the elastomeric component. Stiffness and damping characteristic reaching a predetermined threshold may indicate end of life of the elastomeric component.
12. Payne Effect: Observed in particle-filled elastomers, the modulus at small strains is much higher than at larger strains in dynamic tests. This is attributed to the breakage of the filler network at small strains, leading to a significant drop in modulus. Changes of modulus can be determined by monitoring the remanent magnetic field of the elastomeric component. Reaching a predetermined threshold indicates a bad state of health of the elastomeric component.

The method allows to link strain to a measurable remanent magnetic field of the elastomeric component. The magnitude of the remanent magnetic field and its change is tunable by the selection of the magnetic particles.

Several stress-strain behaviours relate to aging, wear, environmental conditions, etc. These are all parameters that can be attributed to the state of health of the elastomer material and can be used to estimate the end of life of an elastomeric component.

Measuring the remanent magnetic field of the elastomeric component offers a much more comprehensive view on the material behaviour, state and performance than other methods of the prior art, because any parameter which alters the visco-elastic behaviour of the elastomer may be related to the behaviour of the remanent magnetic field through the coupling mentioned before.

In addition, based on the time measurements of the magnetic properties of step c. a temperature effect on magnetism influenced by the magnetic particles or structural changes of the magnetic particles within the elastomer matrix due to humidity may be detected and correlated with the state of health and/or the proper functioning of the elastomeric component.

There are ways to distinguish between response to temperature and a response to strain. The response to temperature is linear, highly repeatable and would be slower than the response to strain. For example: The effect of temperature is a slow phenomenon whereas for example a pumping valve will give a clear distinctive oscillating pattern. The temperature effect will lead to an average of the oscillating pattern slowly shifting.

A plurality of magnetic sensor elements may be used. Depending on the design of the elastomeric component and the device where it is used in, temperature changes may be distributed homogenously whereas changes in strain are not (or vice versa). These differences may be observed by the plurality of magnetic sensor elements.

The elastomeric component may have sections with different formulations having different thermal properties, different mechanical properties, different magnetic properties or combinations thereof. For example, an elastomeric component may have sections with different thermal properties but identical mechanical properties. Strain will induce the same changes in the magnetic properties whereas temperature does not.

An arrangement with the elastomeric component may comprise temperature sensors to correct a change in magnetic properties based on temperature changes. E.g. a digital hall sensor may have an embedded temperature sensor.

Thus, the method provides a solution for:
1. Monitoring the deformation/strain or temperature of the elastomer material in-situ.
2. Monitoring the health, wear (of non-abrasive nature), performance of elastomer components over time and in-situ.
3. Assessing the state of health and predicting the remaining useful life of the elastomeric component.
4. Provide objective measurements of factors related to the degradation of elastomer components and thereby their ability to fulfil their primary critical function e.g. sealing.
5. Allows for the closest measure to the state of health of the material as the particles are part of the material embedded inside the matrix and bonded to it.
6. Allowing to optimize elastomer component design and material selection by measuring directly the stresses and their evolution over time in-situ and as close as possible to the actual application of the elastomeric component.
7. Allowing for "wireless" and non-invasive measurement of relevant mechanical performance parameters of the elastomeric component.

Further embodiments of the invention are set forth in the dependent claims.

In some embodiments the elastomeric component may be mounted in a device for the intended and functional arrangement of the elastomeric component. The magnetic sensor element may be positioned on or in the device. Thus, the measurement of the mechanical property of the elastomeric component can be performed in-situ and while the device is in working condition. The elastomeric component may be e.g. a seal, a membrane, a piston, a protective encapsulation or a damper. The device for the intended and functional arrangement of the elastomeric component may be e.g. a sealed container, an electrochemical cell or pack, a valve, a pump or a bearing.

In some embodiments the magnetic sensor element may comprise a Hall-sensor, preferably a 3D Hall-sensor, or at least one planar inductor coil, preferably several planar inductor coils arranged in a plane.

In some embodiments the magnetic particles may be homogenously dispersed in the entire elastomeric component or in at least one section of the elastomeric component. The at least one section of the matrix may be arranged in a functional section of the elastomeric component.

### Examples of applications:

The elastomeric component may be a seal or gasket in a housing. The magnetic sensor element may be placed in a part of the housing near the seal. The above method may be used to ensure that the expected environmental behaviour, sealing behaviour, mechanical behaviour is not lost when the component is in use.

Although the application of the elastomeric component in a device may be static, the elastomer matrix itself is not, because the internal structure of the elastomer matrix is subject to microscopic deformations and interactions at the molecular level, such as thermal fluctuations, creep, and stress relaxation, which can lead to changes in its mechanical properties over time. Since the magnetic particles are bound to the matrix, they also experience these same microscopic deformations and interactions which in turn leads to a change in the measurable remanent magnetic field out of the component. Comparing the remanent magnetic field right after initial placement of the seal with measurements over time correlates with mechanical properties such as stress relaxation or creep. Thus, the method may be e.g. used to detect e.g. fatigue of the seal.

The elastomeric component may be a vibration isolation or damper arranged in a mount or bushing. Measuring a change in the remanent magnetic field of the vibration isolation or damper over time may be correlated with Mullins effect, hysteresis, stress relaxation, creep, frequency dependency, Payne effect, etc. A key metrics for a vibration isolation may be for example frequency dependency. A vibration isolation must be able to dampen some specific frequency that goes through it, which will show in the magnetic field. Significant change may indicate that the elastomeric component loses its damping function.

The elastomeric component may be a membrane of a membrane pump. By measuring the remanent magnetic field, the performance i.e. correct functioning of the membrane can be directly monitored. The remanent magnetic field follows the pumping cycles. Decrease of the amplitude may indicate decrease of the performance of the membrane, which correlates to the state of health and the remaining time of life.

The intensity of the remanent magnetic field and the magnitude of change depends on the type of magnetic particles used, the filling grade of the magnetic particles and the geometry of the elastomeric component or the section of the elastomeric component comprising the magnetic particles.

In some embodiments the matrix of elastomer material of the elastomeric component may be a thermoset elastomer or a thermoplastic elastomer (TPE). The elastomeric material can be, for example, a synthetic or natural rubber, such as butyl rubber, isoprene rubber, butadiene rubber, halogenated butyl rubber (e.g., bromobutyl rubber), ethylene propylene terpolymer, silicone rubber, fluoro- or perfluoroelastomers, chlorosulfonate, polybutadiene, butyl, neoprene, 30 nitrile, polyisoprene, buna-N, copolymer rubbers such as ethylene-propylene (EPR), ethylene-propylene-diene monomer (EPDM), acrylonitrile-butadiene (NBR or HNBR) and styrene-butadiene (SBR), blends such as ethylene or propylene-EPDM, EPR, or NBR, combinations thereof. The term "synthetic rubbers" also should be understood to encompass materials which alternatively may be classified broadly as thermoplastic or thermosetting elastomers such as polyurethanes, silicones, fluorosilicones, styrene- isoprene-styrene (SIS), and styrene-butadiene-styrene (SBS), as well as other polymers which exhibit rubber-like properties such as plasticized nylons, polyolefins, polyesters, ethylene vinyl acetates, fluoropolymers, and polyvinyl chloride. Good results may be achieved with ethylene propylene diene mono rubber (EPDM), silicone rubber (SR), liquid silicone rubber (LSR), butyl rubber, isoprene or nitrile rubber. The elastomeric material can be chosen to achieve specific strain-stress curves or provide additional properties such as the ability to respond to humidity or temperature changes.

In some embodiments the magnetic particles may be based on magnetic material with high remanence allowing permanent magnetization and may comprise rare earth materials such as neodymium (Nd), niobium (Nb) or samarium (Sm), or more common materials such as boron (B), iron (Fe), cobalt (Co), nickel (Ni) or silicon (Si), or hard ferrites such as strontium ferrite or barium ferrite, or iron-based alloys with high degree of remanence, or mixtures of any of these. The mixture of the materials can be chosen to obtain the desired range of remanence, coercivity, temperature resilience and/or chemical resilience.

In some embodiments the magnetic particles may have an average particle size, which blends homogenously with the matrix without disturbing the desired mechanical properties. Good results have been achieved with an average particle size below 5 microns.

In some embodiments the magnetic particles can be isotropic or anisotropic and accordingly may be dispersed in an isotropic or an anisotropic way.

The invention further relates to a system for monitoring the state of health and/or correct functioning of an elastomeric component according the above-described method.

The system comprises an elastomeric component, a magnetic sensor element for measuring magnetic properties and a processing unit. The elastomeric component comprises a matrix of elastomer material and homogenously dispersed magnetic particles. The processing unit is configured to measure magnetic properties of the elastomeric component with the dispersed magnetic particles via the magnetic sensor element. The processing unit is further configured to determine mechanical properties of the elastomeric component related to strain of the elastomer matrix, a temperature effect on magnetism influenced by the magnetic particles or structural changes of the magnetic particles within the elastomer matrix due to humidity based on the measurements of the magnetic properties and correlate the mechanical properties with the state of health and/or the proper functioning of the elastomeric component.

A measurement of the magnetic properties of the elastomeric component can be achieved in two ways.

Firstly, the dispersed magnetic particles may be permanently magnetized to create a remanent magnetic field in the elastomeric component and the sensor element may measure the remanent magnetic field of the elastomeric component.

Secondly, the magnetic sensor element may create a magnetic field and measure the interactions of the magnetic particles with the generated magnetic field. The magnetic field may be pulsed for continuous measurements.

In some embodiments the elastomeric component of the system may be mounted in a device for the intended and functional arrangement of the elastomeric component. The magnetic sensor element may be positioned on or in the device. Thus, the measurement of the mechanical property of the elastomeric component can be performed in-situ and while the device is in working condition. The elastomeric component may be e.g. a seal, a membrane, a piston, a protective encapsulation or a damper. The device for the intended and functional arrangement of the elastomeric component may be e.g. a sealed container, an electrochemical cell or pack, a valve, a pump or a bearing.

The invention further relates to an arrangement for performing the above-described method. The arrangement comprises an elastomeric component, a magnetic sensor element for measuring magnetic properties and a processing unit connected to the magnetic sensor element. The elastomeric component comprises a matrix of elastomer material and homogenously dispersed magnetic particles. The magnetic sensor element is positioned near the elastomeric component. The processing unit is configured to measure magnetic properties of the elastomeric component with the dispersed magnetic particles via the magnetic sensor element. The processing unit is further configured to determine mechanical properties of the elastomeric component related to strain of the elastomer matrix, a temperature effect on magnetism influenced by the magnetic particles or structural changes of the magnetic particles within the elastomer matrix due to humidity based on the measurements of the magnetic properties and correlate the mechanical properties with the state of health and/or the proper functioning of the elastomeric component.

A measurement of the magnetic properties of the elastomeric component can be achieved by two ways.

Firstly, the dispersed magnetic particles may be permanently magnetized to create a remanent magnetic field in the elastomeric component and the sensor element may measure the remanent magnetic field of the elastomeric component.

Secondly, the magnetic sensor element may create a magnetic field and measure the interactions of the magnetic particles with the generated magnetic field. The magnetic field may be pulsed for continuous measurements.

The arrangement may be a sealing arrangement, a damping arrangement, a pumping arrangement, a dosing arrangement, a sensing arrangement, an actuation arrangement or an insulation arrangement.

### Brief Explanation of the Figures

The invention is described in greater detail below with reference to embodiments that are illustrated in the figures. The figures show:
- Fig. 1: graphic representation of measurement - remanent magnetic field vs time;
- Fig. 2: graphic representation of measurement - detail of Fig. 1;
- Fig. 3: data of a cyclic test measurement with the first 4000 cycles at 0,75 Hz: (a) strain vs force, (b) strain vs magnetic field, (c) force vs magnetic field;
- Fig. 4: data of a cyclic test measurement comparing cycles 0 to 1000 with cycles 37000 to 40000: (a) strain vs force, (b) strain vs magnetic field, (c) force vs magnetic field;
- Fig. 5: data of a cyclic test measurement comparing cycles at different frequencies: (a) strain vs force, (b) strain vs magnetic field, (c) force vs magnetic field;
- Fig. 6: an example of an arrangement of the elastomeric component;
- Fig. 7: an example of an elastomeric component;
- Fig. 8: a further example of an elastomeric component.

### Embodiments of the Invention

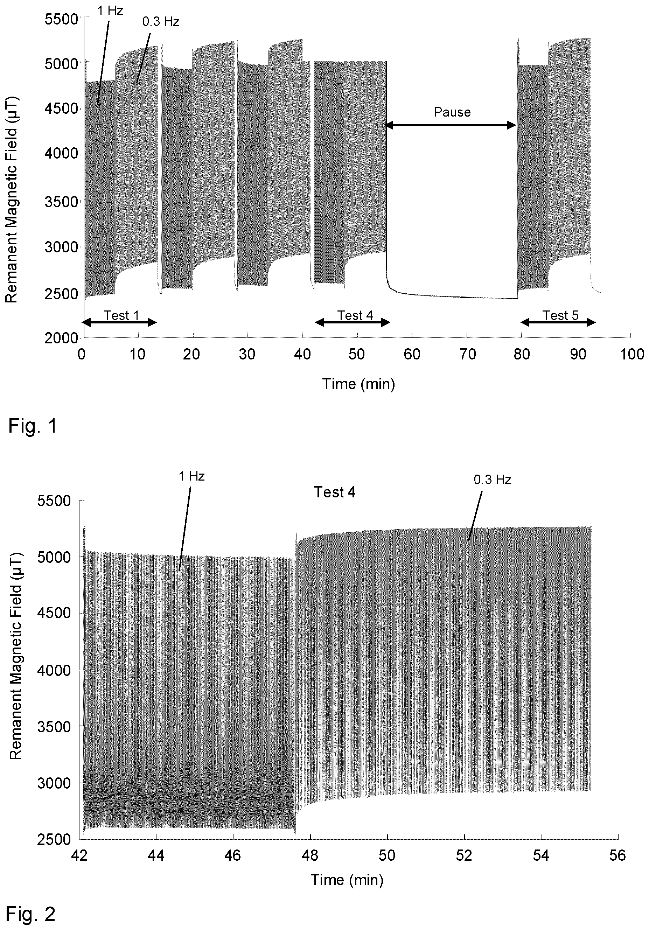

Figs. 1 to 5 show the magneto-mechanical characterization of an elastomeric component. The elastomeric component comprises a matrix of elastomer material and homogenously dispersed magnetic particles, which are permanently magnetized to create a remanent magnetic field. The elastomeric component is placed in a test device for analysing force and strain behaviour of the elastomeric component. A magnetic sensor element for measuring the remanent magnetic field is positioned near the elastomeric component.

Fig 1 shows a graphic representation of a test measurement. The test procedure includes applying a force of up to 50 N to the elastomeric component at 1 Hz for about 200 cycles followed by 0.3 Hz for about 200 cycles over a 1 cm cylinder with 6 mm height. The tests were repeated 5 times with a pause of about 20 minutes between test 4 and test 5. Fig. 2 shows the measurements of Fig. 1 for test 4.

The graphs show the remanent magnetic field (in µT, micro Tesla) versus time (in minutes). The measurement clearly demonstrates a change in remanent magnetic field when a force is applied to the elastomeric component. The measurements further show a dependency on the frequency of actuation and that also shows some typical elastomer behaviour such as the frequency dependency of stress strain. The measurements also show the Mullin's effect which is more dominant at lower frequencies and shows that the material is stabilizing after some time.

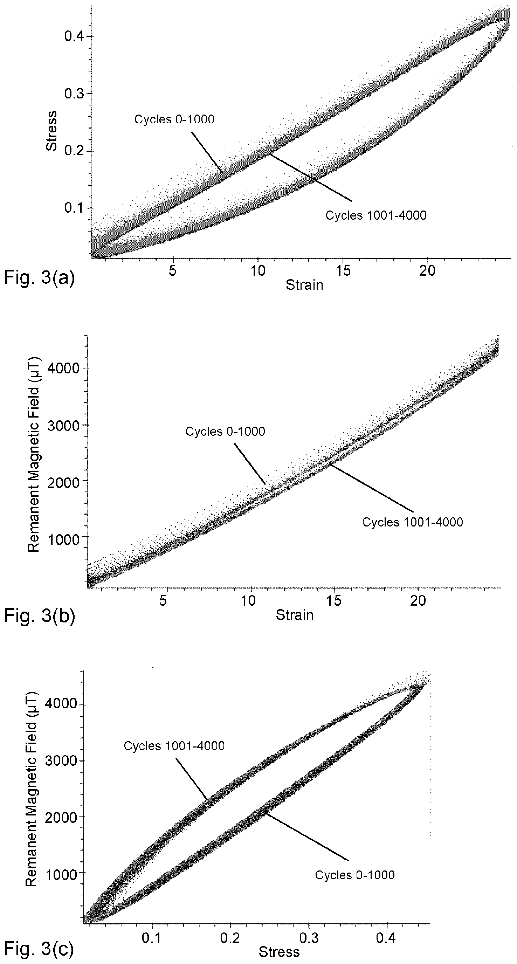

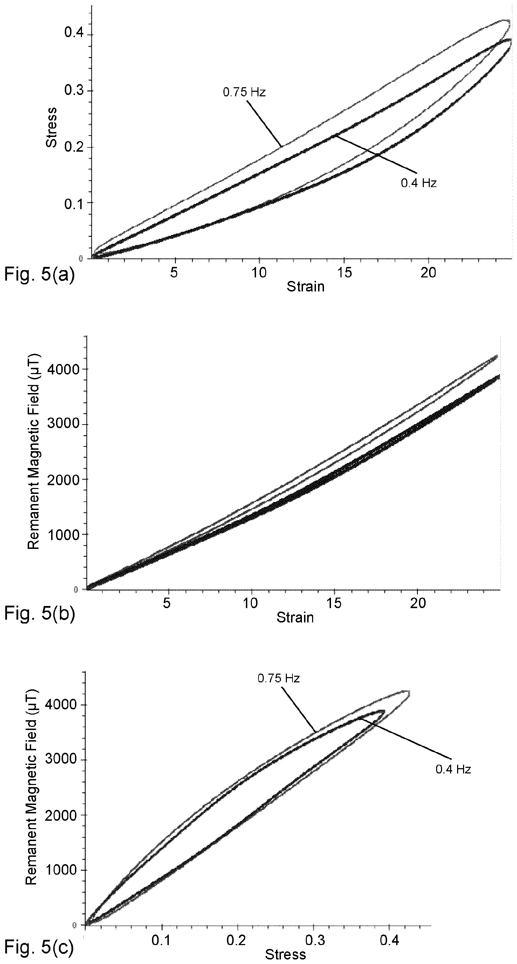

Figs 3 to 5 show data of a cyclic test measurement of an elastomeric component (cylinder with diameter of 10mm and heights of 6 mm) with a target strain of 25% and 40'000 cycles at 0.75 Hz (Figs. 3 to 5) and at 0.4 Hz (Fig. 5). Under (a) strain (in % over 6 mm height) versus stress (in MPa over 80 mm^2), under (b) strain (in % over 6 mm height) versus magnetic field (in µT), and under (c) force (in MPa over 80 mm^2) versus magnetic field (in µT).

The graphs in Fig. 3 show a comparison of cycles 0 to 1000 (scattered points) of a fresh sample with subsequent cycles 1000 to 4000 (lines with different shading). A stabilization in the stress strain hysteresis curve can be observed, which can be attributed to several mechanical properties of an elastomer, mainly Mullins effect (Fig. 3(a)). This same stabilization is observable through the strain-magnetic field curve (Fig. 3(b)) and a stress-magnetic field relationship (Fig. 3(c)). For the measurements, a 25% strain back and forth was applied over an elastomeric component in the form of cylinder of 6 mm height and 10mm diameter for 40'000 cycles at 0.75 Hz.

The measurements clearly demonstrate that a measurement of a remanent magnetic field originating from magnetized homogenously dispersed particles directly relates to mechanical properties of the elastomer matrix.

The hysteresis loop width in the strain-magnetic field relationship is significantly lower than the in the stress-magnetic field relationship. The observation is indicative of stronger coupling in strain-magnetic relationship than in stress-magnetic field relationship.

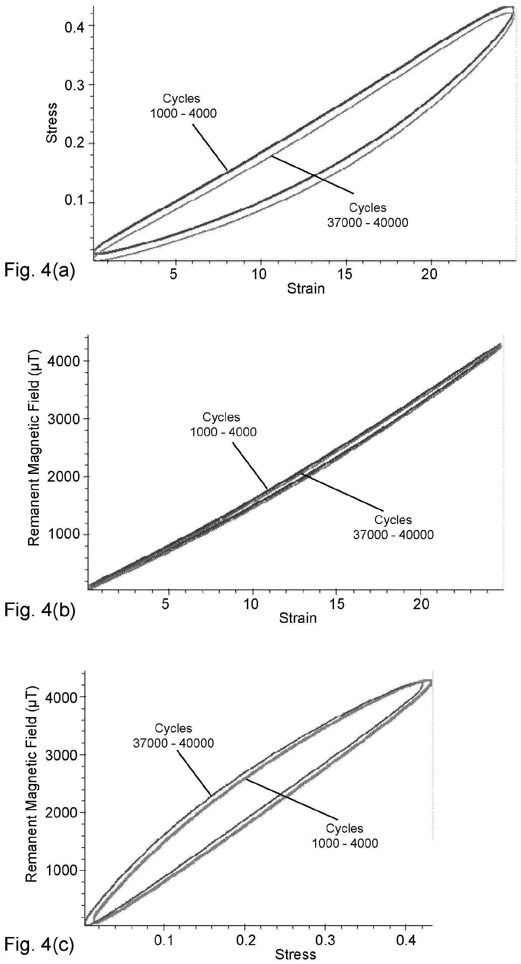

The graphs in Fig. 4 show a comparison of early cycles 1000 to 4000 with cycles 37000 to 40000. A clear softening of the material with a downward shift in the strain stress hysteresis curve can be observed (Fig. 4(a)), which is a typical elastomer behaviour under repeated cyclic load. This can be attributed to multiple phenomena, mainly Mullins effect but also permanent residual deformation over time. This same phenomenon is observable in the strain-magnetic field relationship (Fig. 4(b)) and a stress-magnetic field relationship (Fig. 4(c)). For the measurements, a 25% strain back and forth was applied over an elastomeric component in the form of cylinder of 6 mm height and 10mm diameter for 40'000 cycles at 0.75 Hz.

The graphs in Fig. 5 show a comparison of middle cycles 17000 to 20000 at 0.75 Hz with middle cycles 17000 to 20000 at 0.4 Hz. For the measurements, a 25% strain back and forth was applied over an elastomeric component in the form of cylinder of 6 mm height and 10mm diameter for 40'000 cycles at 0.75 Hz and at 04.Hz. The graphs show that the material at lower frequencies behaves softer whereas it behaves stiffer at higher frequency. This effect relates to the visco-elastic behaviour of elastomers specifically the frequency dependence of strain-stress hysteresis curves. This is observable also from the strain-magnetic field curve.

All measurements of Figs. 3 to 5 clearly demonstrate that a measurement of a remanent magnetic field originating from magnetized homogenously dispersed particles directly relates to mechanical properties of the elastomer matrix. The hysteresis loop width in the strain-magnetic field relationship is significantly lower than the in the stress-magnetic field relationship. The observation is indicative of stronger coupling in strain-magnetic relationship than in stress-magnetic field relationship.

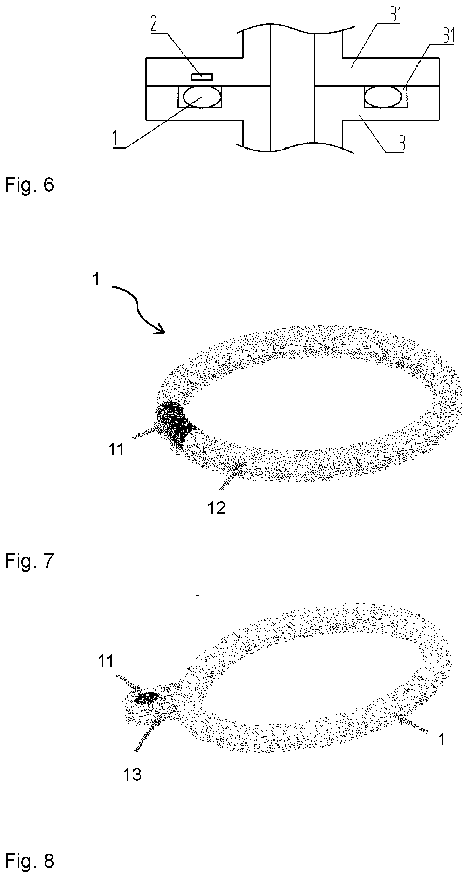

Fig. 6 shows an example of an arrangement for monitoring a mechanical property of an elastomeric component 1 in-situ. The elastomeric component comprises a matrix of elastomer material and homogenously dispersed magnetic particles, which are permanently magnetized to create a remanent magnetic field. The elastomeric component 1 in the shown arrangement is an O-ring placed in a groove 31 of a flange 3 for connecting two tubes. A magnetic sensor element 2 for measuring the remanent magnetic field is placed in the other flange 3' near the O-ring 1.

The magnetic particles may be dispersed and/or magnetized over the entire elastomeric component 1 or only in a section 11 of the elastomeric component, whereas another section 12 is free of magnetized particles. Fig. 7 shows the elastomeric component 1 in the form of an O-ring with only a localized area or section comprising magnetized particles.

Fig. 8 shows an example where the elastomeric component 1 in the form of an O-ring is provided with a small extension 13 comprising a section 11 with the magnetized particles.

Typically, the section of the elastomeric component 1 which is provided with magnetized particles experiences some stress either static or dynamic over a long period of time.

### Reference Signs

- 1: elastomeric component
- 11: section with magnetized particles
- 12: section without magnetized particles
- 13: small extension
- 2: magnetic sensor element
- 3, 3': flange
- 31: groove

## Claims

1. Method for monitoring the state of health and/or correct functioning of an elastomeric component, the method comprising the steps of:
a. providing the elastomeric component, the elastomeric component comprising a matrix of elastomer material and homogenously dispersed magnetic particles;
b. providing a magnetic sensor element for measuring magnetic properties of the elastomeric component with the dispersed magnetic particles, wherein the sensor is positioned in or near the elastomeric component;
c. measuring magnetic properties of the elastomeric component with the dispersed magnetic particles;
d. based on the measurements of the magnetic properties of step c.:
i. determining mechanical properties of the elastomeric component related to strain of the elastomer matrix, a temperature effect on magnetism influenced by the magnetic particles or structural changes of the magnetic particles within the elastomer matrix due to humidity;
ii. correlating the mechanical properties with the state of health and/or the proper functioning of the elastomeric component.

2. Method according to claim 1, wherein the dispersed magnetic particles are permanently magnetized to create a remanent magnetic field in the elastomeric component and the sensor element measures the remanent magnetic field of the elastomeric component.

3. Method according to claim 1, wherein the magnetic sensor element creates a magnetic field and measures the interactions of the magnetic particles with the generated magnetic field.

4. Method according to one of the preceding claims, wherein the mechanical property of the elastomeric component is selected from the group of elasticity, compression set, resilience, fatigue, creep, temperature stability, chemical resistance, mullins effect, hysteresis, stress relaxation, frequency dependence, and payne effect.

5. Method according to one of the preceding claims, wherein the elastomeric component is mounted in a device for the intended and functional arrangement of the elastomeric component and the magnetic sensor element is positioned on or in the device.

6. Method according to one of the preceding claims, wherein the magnetic particles are homogenously dispersed in the entire elastomeric component or in at least one section of the elastomeric component.

7. A system for monitoring the state of health and/or correct functioning of an elastomeric component according to the method of one of the preceding claims, the system comprising an elastomeric component, a magnetic sensor element for measuring magnetic properties and a processing unit,
wherein the elastomeric component comprises a matrix of elastomer material and homogenously dispersed magnetic particles,
wherein the processing unit is configured to measure magnetic properties of the elastomeric component with the dispersed magnetic particles via the magnetic sensor element, and
wherein the processing unit is configured to determine mechanical properties of the elastomeric component related to strain of the elastomer matrix, a temperature effect on magnetism influenced by the magnetic particles or structural changes of the magnetic particles within the elastomer matrix due to humidity based on the measurements of the magnetic properties and correlate the mechanical properties with the state of health and/or the proper functioning of the elastomeric component.

8. System according to claim 7, wherein the dispersed magnetic particles are permanently magnetized to create a remanent magnetic field in the elastomeric component and the sensor element measures the remanent magnetic field of the elastomeric component, or the magnetic sensor element creates a pulsed magnetic field and measures the interactions of the magnetic particles with the generated magnetic field.

9. An arrangement for performing the method according to one of the claims 1 to 6, wherein the arrangement comprises an elastomeric component, a magnetic sensor element for measuring magnetic properties and a processing unit connected to the magnetic sensor element,
wherein the elastomeric component comprises a matrix of elastomer material and homogenously dispersed magnetic particles,
wherein the magnetic sensor element is positioned near the elastomeric component, wherein the processing unit is configured to measure magnetic properties of the elastomeric component with the dispersed magnetic particles via the magnetic sensor element, and
wherein the processing unit is configured to determine mechanical properties of the elastomeric component related to strain of the elastomer matrix, a temperature effect on magnetism influenced by the magnetic particles or structural changes of the magnetic particles within the elastomer matrix due to humidity based on the measurements of the magnetic properties and correlate the mechanical properties with the state of health and/or the proper functioning of the elastomeric component.

10. Arrangement according to claim 9, wherein the dispersed magnetic particles are permanently magnetized to create a remanent magnetic field in the elastomeric component and the sensor element measures the remanent magnetic field of the elastomeric component, or the magnetic sensor element creates a pulsed magnetic field and measures the interactions of the magnetic particles with the generated magnetic field.

11. Arrangement according to claim 9 or 10, wherein the arrangement is a sealing arrangement, a damping arrangement, a pumping arrangement, a dosing arrangement, a sensing arrangement, an actuation arrangement or an insulation arrangement.
